# EUROPÄISCHE PATENTANMELDUNG

(11) **EP 1 693 344 A1**
(43) Veröffentlichungstag der Anmeldung: **23.08.2006**
(21) Anmeldenummer: 06003328.9
(22) Anmeldetag: 17.02.2006
(51) Int. Cl.: C02F 1/02, E03B 7/09, A61L 2/04

(54) **Vorrichtung zur thermischen Desinfektion von Trinkwarmwasserversorgungsanlagen**

(30) Priorität: 18.02.2005 DE 102005007452
(71) Anmelder: Sirius 12 GmbH, 59872 Meschede (DE)
(72) Erfinder: Haase, Georg, 57399 Brachthausen (DE)
(74) Vertreter: Pürckhauer, Rolf

(57) **Zusammenfassung**

Es handelt sich um eine Vorrichtung zur thermischen Desinfektion von Trinkwarmwasserversorgungsanlagen. Um Legionellenwachstum in Trinkwarmwasserversorgungsanlagen mit geringsten technischen Mitteln bei geringstem Energieeinsatz dauerhaft zu verhindern und sie ständig zu desinfizieren, ohne daß dadurch die Nutzung erneuerbarer Energien und Abwärme aus anderen Prozessen behindert oder vermindert wird, und ohne daß dadurch größere Energiekosten und Verluste entstehen, ist in eine Zirkulationsleitung (5) mit Umwälzpumpe (6) ein Durchlauferhitzer (7) eingebaut, der mittels elektrischer oder anderer Energie einen durch ein Regulierventil (8) oder die drehzahlgeregelte Umwälzpumpe (6) eingestellten, geringen Volumenstrom in kurzer Zeit auf eine Temperatur erwärmt, bei der Legionellen absterben.

## Beschreibung

Die Erfindung bezieht sich auf eine Vorrichtung zur thermischen Desinfektion von Trinkwarmwasserversorgungsanlagen.

Trinkwasser ist das wichtigste Lebensmittel überhaupt, und deshalb ist ein neues Denken in der Wasserversorgung erforderlich, besonders seit Inkrafttreten der Trinkwasserverordnung 2001 (TrinkwV 2001) am 1. Januar 2003. Laut Kommentar zum DVGW-Arbeitsblatt W 551 haben Planer sowie Heizungs- und Sanitärinstallateure zwar oft Kenntnisse über Leitungsmaterialien, deren Verbindungstechniken und Korrosionsverhalten, doch fehlt meist das Wissen über Legionellen und deren Wesen sowie Vermehrung derselben in Wassersystemen.

Legionellenpneumonie, die noch sehr oft mit Lungenentzündung verwechselt wird, entsteht, wenn Legionellen direkt in die Lunge gelangen können. Dies geschieht vorwiegend durch Einatmen von Aerosol genannten nebelartigen feinsten Wassertröpfchen, die z.B. beim Duschen entstehen, aber auch schon beim normalen Auslaufhahn im Sonnenlicht zu erkennen sind.

Da in Europa die Meldungen der Pneumoniefälle ständig zunehmen, ist eine Legionellenprophylaxe in der wasserführenden Haustechnik zwingend erforderlich, deren Installation und Betrieb grundsätzlich nach allgemein anerkannten Regeln der Technik und den DVGW-Arbeitsblättern W 551 und W 553 vorzunehmen ist.

Kleinanlagen nach W 551 sind Anlagen mit Speicher-Trinkwassererwärmern oder zentralen Durchfluß-Trinkwassererwärmern in Ein- und Zweifamilienhäusern, unabhängig vom Inhalt der Trinkwassererwärmer und der nachgeschalteten Rohrleitung. Kleinanlagen sind auch die mit Trinkwassererwärmern < 400 Litern und < 3 Litern in jeder Rohrleitung ab Ausgang Trinkwassererwärmer bis zur Entnahmestelle. Empfohlen wird am Warmwasserausgang eine Mindesttemperatur von 60°C, weniger als 50°C sollten aber in jedem Fall vermieden werden und der Betreiber auf das Gesundheitsrisiko hingewiesen werden. Da das größte Wachstumspotential für Legionellen in Speicher-Trinkwassererwärmern im Bereich von 30°C - 60°C zu erwarten ist und im unteren Speicherbereich überwiegend diese niedrigeren Temperaturen vorliegen, ist diese Anordnung nicht empfehlenswert. Auch wenn die Aufheizung des oberen Bereiches mit einem Heizkessel > 60°C und die Umwälzung des gesamten Inhalts einmal am Tag vorgenommen wird, sollte diese Anordnung nicht gewählt werden, denn die Solarenergienutzung wird erheblich vermindert.

Großanlagen sind alle Anlagen in Wohngebäuden, Hotels, Altenheimen, Krankenhäusern, Sportstätten, Schwimmbädern usw., wenn die Anlagen mit Trinkwassererwärmern > 400 1 und / oder Rohrleitungen zwischen Abgang und Entnahmestelle > 3 1 Inhalt ausgestattet sind. Eine Zirkulationsleitung ist einzubauen. Am Warmwasserausgang muß ständig eine Mindesttemperatur von 60°C vorliegen. Da aber auch einmal am Tag der gesamte Systeminhalt auf > 60°C erwärmt werden muß, ist diese Anordnung nicht empfehlenswert. Die Solarenergienutzung wird hier noch weiter vermindert als bei der Kleinanlage.

Im Kommentar zum DVGW-Arbeitsblatt W 551 wird in einer Graphik dargestellt, wieviel Legionellenkonzentrationen in 4.741 Proben bei verschiedenen Temperaturen gefunden wurden. So lagen die Konzentrationen ab 40°C - 50°C am höchsten, ab 50°C - 60°C nur wenig geringer, erst ab 60°C - 70°C erheblich geringer und erst ab 80°C wurden keine Legionellen mehr gefunden.

Aufgrund der neuen Regelung ist der Inhaber oder sonstige Eigentümer einer Hausinstallation, aus der Trinkwasser an Verbraucher abgegeben wird, gleichzeitig auch Betreiber einer Wasserversorgungsanlage und muß sich der daraus entstehenden gesetzlichen Pflichten bewußt sein. Auf einen umfangreichen Ordnungswidrigkeiten- und Straftatbestandskatalog sei hier hingewiesen, der sich aus der TrinkwV 2001 ergibt. So wird nach §75 Abs. 4 des IfSG mit einem Jahr Freiheits- oder Geldstrafe bestraft, wer fahrlässig oder vorsätzlich kontaminiertes Trinkwasser abgibt. Wird dadurch gar eine Legionellose verursacht, ist dies nach §74 Abs. 4 IfSG mit einer Freiheitsstrafe bis zu fünf Jahren oder mit Geldstrafe zu ahnden.

Die Hausinstallation ist nach den Technischen Regeln für die Trinkwasserinstallation durchzuführen, wie sie in der DIN 1988, Teile 1 - 8 dargestellt sind. Verantwortlich dafür sind der Anschlußnehmer (Eigentümer/Vermieter) und der Benutzer (Mieter, Pächter). Sie sind verantwortlich für den einwandfreien Zustand der Hausinstallation und für eine regelmäßige Wartung. Eine Errichtung oder wesentliche Veränderung darf nur durch das Wasserversorgungsunternehmen oder ein Installationsunternehmen, das im Installateurverzeichnis eines Wasserversorgungsunternehmens eingetragen ist, vorgenommen werden.

Derzeitiger Stand der Technik in Trinkwarmwasserversorgungsanlagen sind einerseits Speicher bis 1.000 1, die allein der Warmwasserbereitung dienen, andererseits Kombipufferspeicher ab 300 l, die der Warmwasserbereitung und der Gebäudeheizung dienen. Erstere sind meist mit unten liegenden Wärmetauschern ausgestattet, die von Heizkesseln, Elektro-Heizungen oder Solaranlagen beladen werden, letztere meist mit oben liegenden Wärmetauschern, die von Heizkesseln beladen werden, sowie unten liegenden Wärmetauschern, die von Solaranlagen oder ähnlichen Energiequellen mit niedrigem Temperaturniveau beladen werden.

Grundsätzlich kann gesagt werden, daß der Anteil des Jahresenergiebedarfs eines Gebäudes, der aus erneuerbaren Energien stammt, mit dem Speichervolumen steigt. Zudem ist der Anteil aus erneuerbaren Energien größer, je niedriger die Systemtemperaturen liegen. So kann ein System mit Temperaturen von nur 40°C - 45°C in vielen Fällen die Gebäudeheizung und auch die Warmwasserbereitung sicherstellen, dessen Wärme größtenteils aus erneuerbaren Energien stammen kann.

Aufgrund der Forderung, das gesamte Warmwassersystem inklusive der Leitungen in Kleinanlagen mindestens einmal täglich über 60°C zu erwärmen, in Großanlagen sogar ständig mindestens 60°C am Ausgang des Trinkwassererwärmers sicherzustellen, um Legionellen abzutöten, muß die Erwärmung in der Regel mit einer Energiequelle höheren Temperaturniveaus erfolgen, was zwangsläufig bei großen Volumina auch mit hohen Kosten und Verlusten verbunden ist. Folglich stehen diese über 60°C aufgeheizten Speichersysteme der anschließenden Nutzung erneuerbarer Energien und Abwärme aus anderen Prozessen nicht mehr oder nur sehr eingeschränkt zur Verfügung, weil deren Temperaturniveau meist niedriger liegt.

Bei sogenannten Kombispeichern, das sind Pufferspeicher mit innenliegenden Trinkwasserspeichern, die in der Regel von Heizkesseln, Elektro-Heizungen usw. nur im oberen Bereich höher erwärmt werden können, ist die Aufheizung des unteren Bereiches auf über 60°C praktisch unmöglich. Es werden zwar sogenannte Legionellenschaltungen verschiedenster Art angeboten, die oft sehr komplexe Rohrsysteme und Apparaturen aufweisen, meist sehr teuer sind und die Nutzung erneuerbarer Energien nachhaltig verhindern.

Bekanntlich fällt bei Temperaturen über 60°C in Warmwassersystemen verstärkt Kalk aus. Da aber die Forderungen der TrinkwV 2001 und der DVGW-Arbeitsblätter W 551 und W 553 eingehalten werden müssen, sind Kalkschutzgeräte oder Kalkwandler schon am Kaltwassereingang sinnvoll und unumgänglich.

Anlagen nach DE 42 35 038 C2 haben aufgrund ihrer Komplexität in der Praxis keine Anwendung gefunden. Darin sollen Brauchwasser-Speicher zugleich als Reaktionsbehälter ausgebildet sein, in denen mehrere parallel geschaltete Ladepumpen einen oder mehrere Reaktionsbehälter beaufschlagen. Ferner soll für eine Desinfektionstemperatur von 65°C eine Reaktionszeit von mindestens 15 Minuten und bei 70°C von 4 Minuten erforderlich sein. Außerdem soll in der Kaltwasserleitung ein drosselbares Mengenregelventil eingebaut sein, das eine Überlastung des Reaktionsbehälters bei größeren Zapfmengen verhindern soll. Heizwassermengen-Regelventile, Wärmeübertrager, Dreiwegeventile, Bypaßleitungen, Zirkulationswasser-Verteilventile und Sicherheits-Regelventile sind weitere Bestandteile dieser sehr komplexen Anordnung.

Der Erfindung liegt die Aufgabe zugrunde, Legionellenwachstum in Trinkwarmwasserversorgungsanlagen mit geringsten technischen Mitteln bei geringstem Energieeinsatz dauerhaft zu verhindern und sie ständig zu desinfizieren, ohne daß dadurch die Nutzung erneuerbarer Energien und Abwärme aus anderen Prozessen behindert oder vermindert wird, und ohne daß dadurch größere Energiekosten und Verluste entstehen.

Diese Aufgabe wird erfindungsgemäß mit den Kennzeichnungsmerkmalen des einzigen Patentanspruchs gelöst.

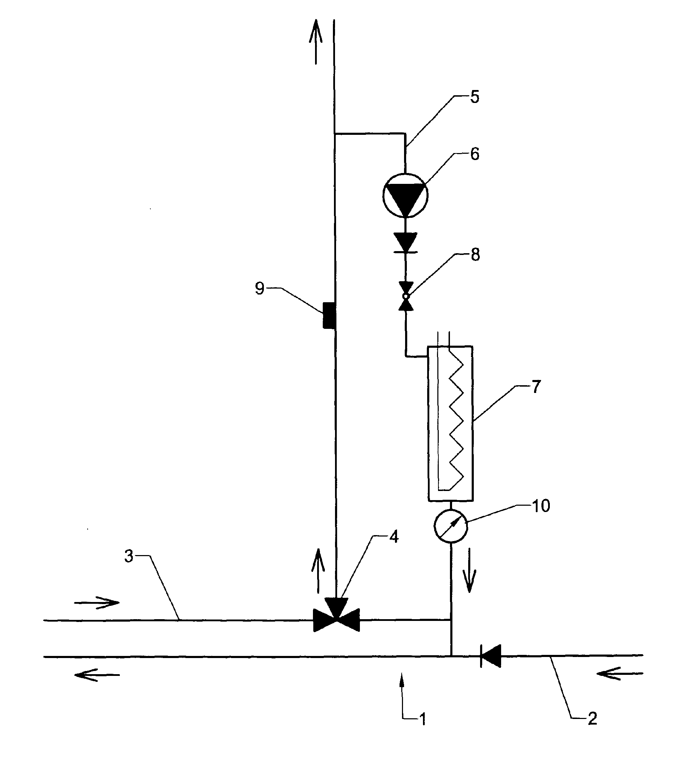

Die erfindungsgemäße Vorrichtung zur thermischen Desinfektion von Trinkwasserversorgungsanlagen zeichnet sich insbesondere durch ihre Einfachheit aus. Es sind lediglich die wenigen in der Zeichnung dargestellten und in der Beschreibung genannten Teile erforderlich. Bei einem Durchfluß von 2 l/min verweilt das Trinkwasser etwa 1 Minute im Durchlauferhitzer. Eine längere Verweildauer ist nicht notwendig, da Legionellen im Laborversuch bei 70°C nur noch einige Sekunden überleben. In 4.741 positiven Proben, die im Stadtgesundheitsamt Frankfurt am Main vorliegen, wurden bei über 83°C keine Legionellen mehr gefunden. Außerdem kann die erfindungsgemäße Vorrichtung kostengünstig in den Handel gelangen und praktisch in jedem Trinkwassersystem in jedem Gebäude, ob in Haushalt, Gewerbe und Industrie, Anwendung finden, sogar auch dort, wo keine klassische Zirkulationsleitung installiert ist.

Weitere Vorteile der Erfindung ergeben sich aus der nachfolgenden Beschreibung anhand eines in der Zeichnung dargestellten Schemas.

In der in dem Schema dargestellten Trinkwarmwasserversorgungsanlage 1 mit einer Kaltwasserzufuhrleitung 2 zum Trinkwassererwärmer (nicht dargestellt), mit einer vom Trinkwassererwärmer wegführenden Warmwasserabgangsleitung 3, mit einem vorgeschriebenen Sicherheits-Thermomischer 4 als Verbrühungsschutz, ist in eine vorhandene Zirkulationsleitung 5 mit einer Umwälzpumpe 6 ein Durchlauferhitzer 7 eingebaut, der mittels elektrischer oder anderer Energie in der Lage ist, einen durch ein Regulierventil 8 oder die drehzahlgeregelte Umwälzpumpe 6 eingestellten, geringen Volumenstrom in kurzer Zeit auf eine Temperatur erwärmt, bei der Legionellen absterben, beispielsweise von 50°C auf über 75°C. Beträgt z.B. die Leistung eines solchen Durchlauferhitzers 7 3,5 kW, ist dieser in der Lage, einen Wasserdurchfluß von 2 1/min von 40°C auf 65°C, oder von 60°C auf 85°C zu erhöhen und damit vorhandene Legionellen und andere Keime abzutöten. Der Durchlauferhitzer 7 wird dabei mit der zeitabhängig gesteuerten Umwälz- bzw. Zirkulationspumpe 6 parallel eingeschaltet und durch einen einstellbaren Thermostaten oder Regler 9 bei beispielsweise 60°C ausgeschaltet, damit der vorgeschriebene Verbrühungsschutz an der Zapfstelle erhalten bleibt. Die Desinfektionstemperatur wird am Thermometer, Regler oder ähnlichem Kontrollgerät 10 überwacht.

Ist der Trinkwassererwärmer ein zentraler Durchfluß-Trinkwassererwärmer in einem größeren Wärmespeicher mit einer Temperatur von 40°C und beträgt sein Inhalt inklusive der Warmwasserleitung z.B. nur 14 Liter, ist dies eine Kleinanlage, in der durch regelmäßige ständige Warmwasserentnahme ein Legionellenwachstum schon aus zeitlichen Gründen erheblich vermindert bis ausgeschlossen ist. Dennoch ist die thermische Desinfektion des Gesamtsystems durch eine Temperaturerhöhung um 25K auf 65°C empfehlenswert und bei einer Leistung von 3,5 kW des Durchlauferhitzer 7 und einem Durchfluß von 2 1/min nach 7 Minuten abgeschlossen. Der Energieeinsatz dafür beträgt nur 0,41 kWh, die dem Wärmespeicher wieder zugeführt werden, der wiederum der kommenden Solarenergienutzung uneingeschränkt zur Verfügung steht. Die thermische Desinfektion des Gesamtsystems kann beliebig wiederholt werden. Auch wenn variable Durchlauferhitzerleistungen und Durchflüsse so eingestellt werden, daß am Thermometer 10 mehr als 100°C gemessen werden, ist der Verbrühungsschutz an der Zapfstelle weiterhin gegeben, weil diese sehr hohe Temperatur nur ab Durchlauferhitzer 7 bis Eingang Trinkwassererwärmer ansteht. Liegt in diesem selbst schon eine Temperatur von über 60°C an, wird der Durchlauferhitzer 7 über den einstellbaren Thermostaten 9 ausgeschaltet, die zeitabhängig gesteuerte Zirkulationspumpe 6 führt den Volumenstrom in diesem Fall nur über den Sicherheits-Thermomischer 4 und nicht durch den Trinkwassererwärmer.

Ist der Trinkwassererwärmer selbst ein Vorratsspeicher in einem größeren Wärmespeicher mit einer Temperatur von 40°C und beträgt sein Inhalt inklusive der Warmwasserleitung zum Beispiel 200 Liter, ist die thermische Desinfektion des Gesamtsystems durch eine Temperaturerhöhung um 25K auf 65°C, bei einer Leistung von 21 kW des Durchlauferhitzers 7 und einem Durchfluß von 20 l/min nach 10 Minuten abgeschlossen. Der Energieeinsatz dafür beträgt nur 5,82 kWh, die dem Wärmespeicher wieder zugeführt werden, der wiederum der kommenden Solarenergienutzung uneingeschränkt zur Verfügung steht. Auch wenn die Leistung des Durchlauferhitzers 7 und Durchfluß so eingestellt werden, daß am Thermometer 10 mehr als 100°C gemessen werden, ist der Verbrühungsschutz an der Zapfstelle weiterhin gegeben, weil diese sehr hohe Temperatur nur ab Durchlauferhitzer 7 bis Eingang Trinkwassererwärmer ansteht. Die thermische Desinfektion des Gesamtsystems kann beliebig wiederholt werden.

Ist keine Zirkulationsleitung 5 vorhanden, werden alle erforderlichen und genannten Teile wie in der Zeichnung dargestellt neu installiert und einerseits am fernsten erreichbaren Punkt der Warmwasserleitung, sowie andererseits am Kaltwassereingang des Trinkwassererwärmers angeschlossen. So ist gewährleistet, daß die Desinfektion des Gesamtsystems auch in sonst nicht erreichbaren unteren Toträumen von Trinkwasserspeichern mit geringsten technischen Mitteln bei geringstem Energieeinsatz unter Beachtung einschlägiger Vorschriften in Alt- wie in Neubauten optimal ablaufen kann und der gleichzeitigen Nutzung erneuerbarer Energien und Abwärme aus anderen Prozessen nichts mehr im Wege steht.

## Patentansprüche

1. Vorrichtung zur thermischen Desinfektion von Trinkwarmwasserversorgungsanlagen, **dadurch gekennzeichnet, daß** in eine Zirkulationsleitung (5) mit Umwälzpumpe (6) ein Durchlauferhitzer (7) mit einer Leistung von z.B. 3,5 kW eingebaut ist, der mittels elektrischer oder anderer Energie die Temperatur eines durch ein Regulierventil (8) oder die drehzahlgeregelte Umwälzpumpe (6) eingestellten Volumenstroms von etwa 2 1/min von 40°C auf 65°C oder von 60°C auf 85°C, d.h. um 25°C erhöht.
